(19) Europäisches Patentamt
European Patent Office
Office européen des brevets

(11) EP 0 993 613 B1

(12) **EUROPÄISCHE PATENTSCHRIFT**

(45) Veröffentlichungstag und Bekanntmachung des Hinweises auf die Patenterteilung:
**12.09.2007 Patentblatt 2007/37**

(21) Anmeldenummer: **98944968.1**

(22) Anmeldetag: **03.07.1998**

(51) Int Cl.:
***G01N 33/53*** *(2006.01)*

(86) Internationale Anmeldenummer:
**PCT/DE1998/001902**

(87) Internationale Veröffentlichungsnummer:
**WO 1999/001764 (14.01.1999 Gazette 1999/02)**

(54) **VERFAHREN ZUR ERKENNUNG UND BESTIMMUNG VON GNRH-REZEPTOREN UND DIE VERWENDUNG VON GNRH-AGONISTEN UND GNRH-ANTAGONISTEN UND ANDERE GNRH REZEPTOR-LIGANDEN ZUR BEHANDLUNG VON TUMOREN MIT GNRH-REZEPTOREN, AUSGEHEND VOM HIRN UND/ODER NERVENSYSTEM UND/ODER DEN HIRNHÄUTEN UND/ ODER VOM KAPOSI-SARKOM**

METHOD FOR RECOGNIZING AND DETERMINING GNRH RECEPTORS AND THE USE OF GNRH AGONISTS AND GNRH ANTAGONISTS AND OTHER GNRH RECEPTOR LIGANDS FOR THE TREATMENT WITH GNRH RECEPTORS OF TUMOURS ORIGINATING IN THE BRAIN AND/OR NERVOUS SYSTEM AND/OR MENINGES AND/OR OF KAPOSI SARCOMA

PROCEDE DE DETECTION ET DE DETERMINATION DE RECEPTEURS GNRH ET UTILISATION D'AGONISTES GNRH, ET D'ANTAGONISTES GNRH, ET AUTRES LIGANDS DU RECEPTEUR GNRH UTILISES DANS LE TRAITEMENT DE TUMEURS A RECEPTEURS GNRH, ISSUES DU CERVEAUX DU SYSTEME NERVEUX OU DES MENINGES, OU POUR TRAITER LE SARCOME DE KAPO

(84) Benannte Vertragsstaaten:
**AT BE CH CY DE DK ES FI FR GB GR IE IT LI LU MC NL PT SE**

(30) Priorität: **04.07.1997 DE 19728737**

(43) Veröffentlichungstag der Anmeldung:
**19.04.2000 Patentblatt 2000/16**

(73) Patentinhaber: **UTI Limited Partnership**
**Calgary, AB T2L 2K7 (CA)**

(72) Erfinder: **Van Groeninghen, Johannes Christianus**
**44287 Dortmund (DE)**

(74) Vertreter: **Grund, Martin**
**GRUND**
**Intellectual Property Group**
**Postfach 44 05 16**
**80754 München (DE)**

(56) Entgegenhaltungen:
**WO-A-90/09799**

- **BIOLOGICAL ABSTRACTS, Philadelphia, PA, USA abstract no. PREV199497360906, siehe Zusammenfassung XP002090711 & J.M. ALEXANDER ET AL.: "Gonadotropin-releasing hormone receptor mRNA expression by human pituitary tumors in vitro." JOURNAL OF CLINICAL INVESTIGATION, Bd. 93, Nr. 6, 1994, Seiten 2332-2339, Boston MA USA**
- **CHEMICAL ABSTRACTS, vol. 124, no. 23, 3. Juni 1996 Columbus, Ohio, US; abstract no. 307632, XP002090712 & S.S. KAKAR ET AL.: "Gonadotropin releasing hormone (GnRH) receptor: molecular cloning, tissue distribution and regulation of gene expression in pituitary, brain and tumor." MOLECULAR ANDROLOGY, Bd. 8, Nr. 1, 1996, Seiten 95-125, Birmingham AL USA**
- **CHEMICAL ABSTRACTS, vol. 129, Columbus, Ohio, US; abstract no. 311053, XP002090713 & J.C. VAN GROENINGHEN ET AL.: "Effects of luteinizing hormone- releasing hormone on nervous system tumors" LANCET, Bd. 352, Nr. 9125, 1. August 1998, Seiten 372-373, London UK**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 993 613 B1

Printed by Jouve, 75001 PARIS (FR)

## Beschreibung

Die vorliegende Erfindung bezieht sich auf die Tumor-Diagnose und -Therapie. Insbesondere bezieht sie sich auf die Diagnose und Therapie von Tumoren, welche GnRH-Rezeptoren tragen. Der GnRH-Rezeptor ist ein wohlbekannter Zielpunkt in der Tumor-Therapie.

Die postoperative Behandlung von Prostata- und Mammakarzinomen mit Agonisten des Gonadotrophin-freisetzenden Hormons (GnRH; in der Literatur gleichgesetzt mit luteinisierendem Hormon-freisetzendern Hormon; LH-RH) ist eine Standardbehandlung; vgl Gonzalez-Barcena et al., 1994, The Prostate 24, 84-92; Emons und Schally, 1994, Human Reproduction Update 9, Nr. 7, 1364-1379.

So ist bei verschiedenen Steroidhormon-(Sexualhormon)-abhängigen malignen Tumore, wie Mammakarzinom, Prostatakarzinom, Ovarialkarzinom, und Endometriumkarzinom in klinischen Studien ein Doppeleffekt bei der Behandlung mit GnRH-Agonisten festgestellt worden:

1) eine indirekte anti-proliferative Wirkung durch Entkoppeln der endokrinen (östrogenen oder androgenen) positiven Beeinflussung des Wachstums des Tumors,
2) eine direkt anti-proliferative Wirkung durch einen unbekannten Mechanismus über GnRH-Rezeptoren im Tumorgewebe selbst; vgl. Emons und Schally, 1994, Human Reproduction Update 9, 1364-1379.

Die vorgenannte indirekte Wirkung aufgrund einer Steroidhormonabhängigkeit ist seit Jahrzehnten für das Prostatakarzinom und Mammakarzinom bekannt; vgl Gonzalez-Barcena et al., 1994, The Prostate 24, 84-92; Jonat et al., 1995, European Journal of Cancer 31A, 137-142.

Die direkte anti-proliferative Wirkung von GnRH-Agonisten und GnRH-Antagonisten auf z.B. Prostatakarzinome, Mammakarzinome und Ovarialkarzinome wurde in klinischen Studien bestätigt. Einige für diese Behandlung eingesetzte GnRH-Agonisten, die eine direkte anti-proliferative Wirkung besitzen, sind unter den folgenden Handelsnamen der in Deutschland zugelassenen Arzneimittel bekannt: z.B. Zoladex®, Zoladex 10,8®, Zoladex Gyn®, Profact®-Depot, Profact pro injectione /-nasal, Synarela®, Enantone Monats-Depot®, Uno-Enantone®, Enantone-Gyn Monats-Depot®, Trenantone®, Suprecur®, Carcinil® oder Decapeptyl® 0,5 mg/0,1 mg, Decapeptyl® Depot, Decapeptyl®Gyn und Decapeptyl® Diagnostik. Ein Beispiel eines in mehreren klinischen Studien erforschten GnRH-Antagonisten ist Cetrorelix®, das in Deutschland noch nicht als Arzneimittel zugelassen ist. Eine Cetrorelix®-Behandlung hat den Nachteil, daß kein Depotpräparat mit z.B. wochenlanger Wirkung existiert. Andere Beispiele von experimentell verwendeten GnRH-Antagonisten sind Antarelix® und Antide®, wobei für Antide® als Ausführungsform auch eine orale Darreichungssform besteht *(*Russel-Jones et al., 1995, Bioconjugate Chem. 6, 34-42*).*

Forschung in Zellkultur zeigte, daß auf humanen primären Leberzellkarzinomen und Pancreasadenokarzinomen GnRH-Rezeptoren vorkommen. Ferner wurde ein Anfang einer biochemischen Metabolisierung im Sinne einer Spaltung von GnRH zwischen Tyrosin 5 und Glycin 6 in Ratten-Glioma und Ratten-Neuroblastoma beschrieben; vgl. Tao et al., 1991, Neuropeptides 20, 125-131. Die Ligand Bindung von GnRH am GnRH-Rezeptor und dessen Signalübermittlung findet jedoch auf andere Weise statt, nämlich an der 8. GnRH-Aminosäure Arginin, und ausschließlich im Falle einer intakten Konformation des GnRH Moleküls und seiner Aminosäure-Seitenketten (Naor, Z., Schacham, Sh., Harris, D., Seger, R., and Reiss, N., 1995, Signal Transduction of the Gonadotropin Releasing Hormone (GnRH) Receptor: Cross-Talk of Calcium, Protein Kinase C (PKC), and Arachnoidonic Acid. Cellular and Molecular Neurobiology, Vol. 15, 527-545). In der normalen Ratte Adenohypophyse, wo GnRH-Rezeptoren sind, verursacht GnRH eine erhöhte cAMP Produktion, aber unklar ist noch, ob dies ein direkter oder indirekter Effekt (parakrine Interaktion) ist. Bei der Funktion des GnRH-Rezeptors in der Ratte, welche eine Sezernierung von LH und eine erhöhte Produktion von LH, stimuliert durch GnRH, beinhaltet, spielt die biochemische Metabolisierung von GnRH, z.B. mittels cAMP, lediglich eine indirekte Rolle (Abdilnour, G. und Bourne, G.A., 1995, Adenosine 3',5'-cyclic mono-phosphate and the self-priming effect of gonadotrophin-releasing hormone. Molecular and Cellular Endocrinology, 107, 1-7). Selbstverständlich wurden auf humanen Gonadotropin produzierenden Hypophysenadenomen GnRH-Rezeptoren festgestellt (Alexander, J.P. und Klibanski, A. Gonadotropin-releasing Hormone Receptor mRNA Expression by Human Pituitary Tumors In Vitro, 1994, Journal of Clinical Investigation, 93, 2332-2339). Für die Behandlung der Indikation Pubertas Praecox, z.B. entstanden durch das GnRH produzierende hypothalame Hamartoma, wurden GnRH-Agonisten zur Symptombehandlung für die Blockierung der Gonadotropin produzierenden Zellen in der Adenohypophyse auch bei Kindern eingesetzt (Mahachoklertwattana, P., Kaplan, S.L., Grumbach, M.M. The Luteinizing-Hormone-Releasing Hormone-Secreting Hypothalamic Hamartoma Is a Congenital Malformation: Natural History, 1993, Journal of Clinical Endocrinology and Metabolism, 77, 118-125).

Für Glioma und andere maligne Tumoren ektodermalen Ursprungs, wie z.B. das maligne Melanom, insbesondere im Falle diffus wachsender Tumore im Nervensystem oder im Falle von Metastasierung (die Bildung von Absiedlungen, z.B. in anderen Organen), ist die Lebenserwartung nicht optimistisch. Dasselbe gilt für das Kaposi-Sarkom. Glioma werden die von der Neuroglia ausgehenden, d.h. vom Ektoderm abgeleiteten Hüll- und Stützgewebe des Nervensystems, vor allem im Gehirn lokalisierten echten Tumoren des zentralen Nervensystems (ZNS) genannt. Diese Glioma treten in unterschiedlicher Differenzierung auf. Unterarten der Glioma sind das Spongioblastom, Oligodendrogliom, Astrozytom, Glioblastom und Retinoblastom. Vor allem der Hirntumortyp des Glioblastoma multiforme (GBM) zeichnet sich durch ein sehr schnelles Wachstum und seine extrem hohe Rezidivrate (d.h. Prozentsatz von Patienten mit Hirntumorrückkehr nach operativer makroskopischer Entfernung) aus.

Sowohl das im ZNS auftretende maligne Melanom, primär oder als Metastase, als auch das primär in der Haut auftretende maligne Melanom und/oder das weiter in der Haut und/oder in anderen Körperorganen sich absiedelnde (metastasierende) maligne Melanom gehören zu den Tumoren, die vom Nervensystem ausgehen; vgl Shamamian et al., 1994, Cancer Immunol. Immunother. 39, 73-83; Florenes et al., 1994, Cancer Research 54, 354-356. Maligne Melanome stammen aus dem Neuroektoderm, einer embryonalen Anlageschicht. Burg et al., 1997, Deutsches Ärzteblatt 94, 890-895, beschreiben einen tumorwachstumshemmenden Effekt von Tamoxifen für das maligne Melanom. Ferner besitzen Glioblastoma und malignes Melanom gemeinsam verschiedene Tumormarker; vgl. Shamamian et al., 1994, Cancer Immunol. Immunother. 39, 73-83; Florenes et al., 1994, Cancer Research 54, 354-356. Die Prognose ist im Fall einer Metastasierung sehr schlecht; vgl. Burg et al., 1997, Deutsches Ärzteblatt 94, 890-895.

Zu den Tumoren, die vom Hirn und/oder Nervensystem und/oder den Hirnhäuten ausgehen, zählen ferner das Neuroblastom und das Medulloblastom, in ihrer Gesamtheit klassifiziert unter den sogenannten primitiven neuroektodermalen Tumoren, abgekürzt PNET. Weiter gehören zu diesen Tumoren das Pinealom, ausgehend vom Pinealis (Zirbeldrüsen)-Parenchym und/oder von primordialen Keimzellen im Pinealisbereich oder in der Hirnmittellinie. Ferner ist bei der Zirbeldrüse der Ursprung für das Craniopharyngeom (ein β-HCG bzw. LH-ähnliches Glycoprotein produziernder Tumor; vgl. Tachibana et al., 1994, J. of Neurosurgery 80, 79-84) gelegen, das zu den ektodermalen Tumoren gerechnet wird und von der Vorder-/Oberseite der Hypophyse ausgeht.

Sowohl für das Craniopharyngeom als auch das Meningeom, das als ein gutartiger Tumor ausgehend von Arachnoidaldeckzellen angesehen wird und oft fest an der Innenseite der Hirnhaut (Dura mater) haftet, wurden Progesteron- und Östrogen-Rezeptoren beschrieben. Ferner wurden Androgen-Rezeptoren für das Meningeom nachgewiesen. In klinischen Studien mit Anti-Progesteron-Arzneimitteln wurden tumorschrumpfende Effekte nachgewiesen.

Die bisherige Erprobung weiterer Therapien (verschiedene Chemotherapieformen, Strahlentherapie, etc.) hat in zahlreichen klinischen Studien keine wesentliche Verbesserung der Prognose für Tumore, die vom Hirn und/oder Nervensystem und/oder den Hirnhäuten ausgehen, erbracht. Zur Zeit besteht die Standardtherapie beim Glioblastoma multiforme nach wie vor aus einer möglichst vollständigen operativen Tumorentfernung und einer anschließenden konventionellen Strahlentherapie. Unter dieser Standardtherapie liegt die statistisch angegebene mittlere Überlebenszeit bei 9-13 Monaten, wobei interindividuelle Schwankungen und insbesondere eine etwas bessere Prognose bei jüngeren Patienten beobachtet wurden.

Ungefähr 30% der Patienten mit rezidivem Glioblastoma multiforme zeigten eine gleichbleibende Größe bzw. ein Schrumpfen des nicht mehr operablen Rest-Himtumors unter andauernder hoher Dosierung mit Tamoxifen®, einem Anti-Östrogen-Präparat. Dieser tumorhemmende Effekt wird bei der Glioblastoma-Behandlung nicht auf seine antiöstrogene Wirkung, sondern auf seine Hemmung der Proteinkinase-C (einem intrazellulären Signalüberträger) zurückgeführt; vgl. Puchner et al., Zentralblatt für Neurochirurgie, Supplement 1996, 47. Jahrestagung Deutsche Gesellschaft für Neurochirurgie, Seite 44; Pollack et al., 1995, The Efficacy of Tamoxifen as an anti-proliferative Agent in vitro for Benign and Malignant Pediatric Glial Tumors, Pediatr. Neurosurgery, 22, 281-288). Ferner soll Tamoxifen® die Empfindlichkeit der Tumorzellen sowohl für platinhaltige Chemotherapeutika als auch für die Strahlentherapie erhöhen.

Für Glioblastoma multiforme (WHO Grad IV Astrozytoma) und für Glioma niedrigen Malignitätsgrades (WHO Grad II-IV Astrozytoma) sind Steroidhormon-Rezeptoren in einem geringen Prozentsatz der Fälle festgestellt worden (vgl. Paoletti et al., 1990, J. Neurosurgery, Characteristics and biological role of steroid hormone receptors in neuroepithelial tumors, 73, 736-742). Eine indirekte anti-proliferative Wirkung bei Glioblastoma multiforme und Glioma Grad II-IV wurde bis jetzt lediglich in etwa 30% der Fälle in klinischen Studien durch Reagieren des Tumors auf Tamoxifen® (ein Anti-Östrogen-Präparat) festgestellt.

Obwohl einige relativ günstige neue Entwicklungen in der Therapie von Glioblastoma multiforme in jüngster Zeit beschrieben wurden, bleibt eine schlechte Prognose quod vitam für Patienten mit Glioblastoma multiforme aufgrund der extrem hohen Rezidivrate trotz bisher erprobter Therapieformen und des Mangels an einer spezifischen gezielten Therapie und Frühdiagnostik weiter bestehen.

Der Erfindung liegt einerseits die Aufgabe zugrunde, Diagnostika bereitzustellen, die Tumoren ausgehend vom Hirn und/oder Nervensystem und/oder den Hirnhäuten und/oder Kaposi-Sarkom bereits im Frühstadium erkennen und andererseits die Aufgabe, ein Arzneimittel für die Therapie dieser Tumoren bereitzustellen, das zu einer für alle Patienten besseren Prognose führt.

Die Erfindung betrifft ein *ex vivo* Verfahren zur Erkennung und Bestimmung von GnRH-Rezeptoren auf Tumorzellen vom Hirn neuroektodermalen Ursprungs und/oder Hirnhäuten und/oder vom malignen Melanom und/oder vom Kaposi Sarkom, umfassend das Kontaktierengenannter Zellen mit einem Ligand für einen GnRH-Rezeptor, und Bestimmen, ob ein Binung stattgefunden hat Weiterhin bettifft die Erfindung die Verwendung von GnRH-Agonisten oder GnRH-Antagonisten zur Herstellung eine Arzneimittels zur Behandlung eines Tumors mit GnRH-Rezeptoren vom Hirn neuroektodermalen Ursprungs und/oder den Hirnhäuten, und/oder zur Behandlung eines malignen Melanoms und/oder zur Behandlung eines Kaposi-Sarkoms.

Die direkte anti-proliferative Wirkung von GnRH-Agonisten auf hirneigenen Tumoren, z.B. Glioblastoma Multiforme, wurde noch nicht beschrieben. Weiterhin war bisher nicht bekannt, daß GnRH-Rezeproren auf humanen ektodermalen Tumoren, wie z.B. Glioblastoma Multiforme, vorkommen. Auch war bisher nicht bekannt, daß GnRH-Rezeptoren auf Kaposi-Sarkom vorkommen.

Die vorliegende Erfindung liefert einen Beitrag zur Verbesserung der Diagnose und Therapie von Tumoren ausgehend vom Hirn und/oder vom Nervensystem und/oder von den Hirnhäuten und/oder vom Kaposi-Sarkon indem ein geeigneter Zielpunkt für die Diagnose und Therapie bereitgestellt wird.

Die Verwendung von Diagnostik-Kits zum Nachweis von GnRH-Rezeptoren zur immunhistologischen Diagnostik, und/oder zum Nachweis von GnRH-Rezeptor mRNA, zur Therapieüberwachung, zur Nachsorge zur Rezidivfrüherkennung während der Nachuntersuchung des immer vorhandenen Residualtumors nach einer Operation z.B. eines Gliomas niedrigen Grades (G II-III WHO; vgl. World Health Organization (WHO) Klassifikation der Tumoren des Zentral- und Periphernervensystems, in: Kleihues et al., 1993, Histological Typing of Tumors of the Zentral Nervous System, Springer Verlag, Berlin-Heidelberg-New York-Tokyo), oder um eine Malignisierung im Sinne eines Glioblastoma multiforme (G IV) festzustellen, und zur Früherkennung in Risikogruppen für eine Durchmusterung auf das Vorkommen von Tumoren z.B. von Glioblastoma multiforme ausgehend vom Hirn und/oder Nervensystem und/oder den Hirnhäuten wird beschrieben.

Der Kit kann zum Nachweis von GnRH-Rezeptoren auf Zellmembranen oder in Körperflüssigkeiten z.B. Blut, Plasma, Serum, Urin oder Hirnwasser, Gewebeextrakten, Gewebeflüssigkeiten, *in vitro*-Zellkulturüberständen und Zell-Lysaten verwendet werden. Der GnRH-Rezeptor kann z.B. immunhistochemisch in z.B. operativ entfernten Tumorpräparaten oder Zellkulturen oder mittels eines üblichen Radio-Immun-Assay in z.B. Körperflüssigkeiten bestimmt werden. Der Diagnostik-Kit umfaßt einen GnRH-Agonisten und/oder einen GnRH-Antagonisten und/oder einen mono- oder polyklonalen Antikörper gegen humane GnRH-Rezeptoren und/oder einen oder mehrere spezifische Primer gegen GnRH-Rezeptoren, z.B. zur Amplifizierung der cDNA eines GnRH-Rezeptors in einer Reverse-Transkriptase-Polymerase-Kettenreaktion (RT-PCR). Der Nachweis von GnRH-Rezeptoren wird in an sich bekannter Weise mit bekannten Immunnachweisen, insbesondere mit Enzym-gebundenen Immunoabsorptionsnachweisen (enzym-linked immunoadsorbent assay; ELISA) oder mit dem hier nachstehend beschriebenen Verfahren zur Erkennung und Bestimmung von GnRH-Rezeptoren auf entarteten Zellen durchgeführt.

Das erfindungsgemäße ex vivo Verfahren zur Erkennung und Bestimmung von GnRH-Rezeptoren auf entarteten Zellen eines Tumors ausgehend vom Hirn und/oder Nervensystem und/oder den Hirnhäuten umfaßt in einer bevorzugten Ausführungsform die folgenden Schritte : a) Homogenisieren von peroperativ gesammeltem Tumorgewebe, b) Abtrennen der Membranfraktion, c) Bestimmen der Proteinkonzentration in der Membranfraktion von b), d) Bestimmen der GnRH-Rezeptorkonzentration in der Membranfraktion von b). Besonders vorteilhaft ist das erfindungsgemäße Verfahren zur Erkennung und Bestimmung von GnRH-Rezeptoren für Gewebe, das von einem Glioblastoma multiforme, Medulloblastom, Pinealom, Neuroblastom, Craniopharyngeom, Meningeom, Chordom, Ewing Sarkom, malignem Melanom oder Kaposi-Sarkom ausgeht. Dieses Verfahren ermöglicht die Diagnose von Tumoren.

In einer besonders bevorzugten Ausführungsform wird frisches, humanes Tumorgewebe z.B. während einer Hirntumoroperation (peroperativ) gesammelt und anschließend in flüssigem Stickstoff aufbewahrt. Für die GnRH-Rezeptorbestimmung werden die gefrorenen Gewebeproben zerkleilnert und homogenisiert. In einem Zentrifugationsschritt werden die Proben von größeren Gewebetrümmern getrennt. Der Überstand wird erneut zentrifugiert. Das erhaltene Sediment (Pellet) enthält die Membranfraktion, die erneut homogenisiert wird, um eine möglichst homogene Membransuspension zu erhalten. Die Membransuspension wird zur GnRH-Rezeptorbestimmung im Radio-Rezeptor-Assay eingesetzt. Zuvor wird in üblicher Weise, z.B. unter Verwendung des Bio-Rad-Protein-Assay (Bio-Rad, München), die Proteinkonzentration in der präparierten Membranfraktion in bekannter Weise photometrisch bestimmt. Die Bestimmung der GnRH-Rezeptorkonzentration erfolgt unter Verwendung eines bekannten GnRH-Agonisten, wie z.B. Buserelin®, der spezifisch an GnRH-Rezeptoren in der präparierten Membranfraktion bindet. Da der GnRH-Agonist radioaktiv markiert ist, z.B. mit $^{125}$J, gibt die Konzentration des gebundenen radioaktiv markierten GnRH-Agonisten die Konzentration der GnRH-Rezeptoren in der Membranfraktion wider. Die Konzentration des gebundenen radioaktiv markierten GnRH-Agonisten wird anhand der radioaktiven Zerfälle pro Minute bestimmt. Es werden somit sowohl die Low Affinity/High Capacity als auch die High Affinity/Low Capacity GnRH Rezeptor Bindungsstellen bestimmt (vgl. BAUMANN, K. et al., 1993, Breast Cancer Research Treatment, Vol. 25, Seite 37-46).

Ferner betrifft die Erfindung die Verwendung von GnRH-Agonisten oder GnRH-Antagonisten zur Herstellung eines Arzneimittels zur Behandlung eines Tumors, mit GnRH-Rezeptoren vom Hirn neuroektodermalen Ursprungs und/oder den Hirnhäuten und/oder zur Behandlung eines malignen Melanoms und/oder zur Behandlung eines Kaposi-Sarkoms. Insbesondere betrifft die Erfindung die Verwendung von GnRH-Agonisten oder GnRH-Antagonisten zur Herstellung eines Arzneimittels zur Behandlung eines Glioblastoma multiforme, Medulloblastoms, Pinealoms, Neuroblastoms, Craniopharyngeoms, Meningeoms, Chordoms, Ewing sarkoms, malignen Melanoms oder Kaposi-Sarkoms. GnRH-Rezeptoren als auch eine GnRH-Agonist/Antagonist-Behandlung sind bisher weder für das Craniopharyngeom noch für das Meningeom, noch für das Chordom, noch für das Ewing Sarkom, noch für das maligne Melanom und auch nicht für dasKaposi-Sarkom beschrieben worden. Eine Blut-Hirn-Schranke ist bei diesen Tumoren nicht vorhanden, da sie vom Ursprung extracerebrale intrakranielle oder periphäre Tumore sind. Daher ist die erfindungsgemäße Therapie mit GnRH-Agonisten und/oder GnRH-Antagonisten bzw. deren Konjugaten vorteilhaft. Die Blut-Hirn-Schranke ist jedoch für GnRH durchgängig, da ein Zwei-Richtungen-System, ein bidirektioneller aktiver Transport von GnRH durch die Blut-Hirn-Schranke besteht (Barrera, C: Banks, W.A., Fasold, M.B. and Kastin, A.J., 1991, Effects of Various Reproductive Hormones on the Penetration of LHRH Across the Blood-Brain Barrier, Pharmacology, Biochemistry & Behaviour, Vol. 41, 255-257). Daher ist die Behandlung mit GnRH-Agonisten/GnRH-Antagonisten vorteilhaft gegenüber der Behandlung mit Tamoxifen, für das eine Blut-Hirn-Schranke besteht. Für das Ewing sarkom oder für andere periphäre Formen von PNET außerhalb des Zentral Nervensystems, für das maligne Melanom und für das Kaposi-Sarkom spielt die Blut-Him-Schranke in der Regel keine wesentliche Rolle bei der Behandlung mit GnRH-Agonisten/Antagonisten, da diese Tumoren meistens außerhalb der Blut-Hirn-Schranke entstehen und bleiben.

Tabelle I:

Liste von GnRH-Agonisten und GnRH-Antagonisten, welche zur Behandlung eines Tumors mit GnRH-Rezeptoren, ausgehend vom Hirn und/oder Nervensystem und/oder Hirnhäuten und/oder vom Kaposi-Sarkom eingesetzt werden können:

| **GnRH-Agonisten:**<br>Pharmakologische Stoffname: | **GnRH-Antagonisten:**<br>Handelsname: |
|---|---|
| Leuprorelinacetat, Leuprorelin | Cetrorelix®, Asta Medica AG, Frankfurt/Main |
| Triptorelinacetat, Triptorelin | Antarelix®, Asta Medica AG, Frankfurt/Main |
| Buserelinacetat, Buserelin | Antide®, Ares Sarono Int. SA, Genève, Schweiz |
| Goserelinacetat, Goserelin | Ramorelix®, Hoe013HoechstGmbH, Frankfurt/M. |

Weitere Beispiele von GnRH-Antagonisten sind:

- LHRH Antagonisten ähnlich wie Antide®, beschrieben in US-Patent 5.480,969 *(Bowers et al., Date of Patent Jan. 2, 1996)*
- LHRH Peptide Derivaten, beschrieben in UK-Patent GB 2 246782 B *(Albert, R. et al., Patent published 16.09.1992)*
- LHRH Antagonisten, beschrieben in US-Patent 5,198,533 *(Schally et al., Date of Patent: Mar. 30, 1993)*

Die Behandlungsdosierung von den GnRH-Agonisten in obenstehender Liste entspricht minimal der in der Roten Liste® für die jeweiligen GnRH-Agonisten angegeben Dosierung für andere Verwendungsindikationen für die subkutane bzw. intramuskulare Verabreichungsform. Für die intravenöse Verabreichung von GnRH-Agonisten wird die minimale Tagesdosierung eingesetzt, vgl. z.B. *Klijn et al., 1982, The Lancet, 1213-1216.*

Die Behandlungsdosierung von den GnRH-Antagonisten Cetrorelix®, Antrarelix®, Antide® und Ramorelix® für die subkutane und intramuskulare Verabreichungsform in obenstehender Liste entspricht minimal der Dosierung, welche z.B. in der Literatur beschrieben wurde und eingesetzt wird bei anderen Indikationen, vgl. z.B. für Cetrorelix®: Gonzalez-Barcena et al., 1994, The Prostate 24, 84-92.

Die Behandlungsdosierung von den GnRH-Agonisten Cetrorelix®, Antarelix®, Antide® und Ramorelix® für die intravenöse Verabreichungsform in obenstehender Liste entspricht minimal der Dosierung, welche für anderen Indikationen bei der betreffenden Zulassungsbehörde bekannt ist, oder in der Deutschen Pharmazeutischen Stoffliste oder in der Literatur beschrieben und gegeben wird, z.B. für Antide® : Fattinger et al., 1996. Am. J. Physiol. 271 (Endocrinol. Metab. 34): E775-E787. Dasselbe gilt auch für die GnRH-Antagonisten beschrieben in US-Patent 5,480,969, CJK-Patent GB 2 246782 B und US-Patent 5,198,533.

Der Erfindung nach können GnRH-Agonisten und/oder -Antagonisten in jeder geeigneten Form verwendet werden. Bei Tumoren innerhalb der Blut-Hirn-Schranke wird die direkte Injektion, z.B. in den Kreislauf, intraarteriell direkt im Nervensystem Kreislauf oder intravenös oder Injektion in den Liquorwegen, oder Applikation örtlich im Tumorbett nach einer Operation, direkt nach makroskopischer Tumorentfernung peroperativ oder mit Ommaya® reservoir oder einer anderen Form der subkutanen, ventrikulären Injektion in den Liquorwegen bevorzugt. Es ist möglich, sowohl GnRH-Agonisten als auch GnRH-Antagonisten zu benutzen, weil beide als Ligand an den GnRH-Rezeptor binden. Ferner sind Liganden, die spezifisch auf den GnRH-Rezeptor zielen, verwendbar, wie vorzugsweise humane oder humanisierte Antikörper. In vielen Fällen ist bevorzugt, sicherzustellen, daß das zielende Agens vornehmlich Tumorzellen erreicht. Deswegen sind bildgebende (Engl. imaging) Verfahren, bei dem Liganden mit Tracern benutzt werden, ein weiterer Aspekt der Erfindung. Wenn der Ligand vornehmlich im Tumor lokalisiert wird, kann der Ligand gekoppelt werden an ein cytotoxisches Agens, wie z.B. ein Radioisotop, oder an eine andere toxische Substanz, wie Ricin A oder dergleichen. Bevorzugte GnRH-Agonisten sind aufgeführt in der Roten Liste, auf die hier ausdrücklich Bezug genommen wird (Rote Liste, 1997, Abschnitt 50, Teil 3, Hypothalamushormone, 50038 bis 50056, Herausgeber ROTE LISTE® Service GmbH, Frankfurt/Main. Bevorzugte GnRH-Antagonisten, welche bereits klinisch an Patienten für andere Behandlungen eingesetzt wurden, sind Cetrorelix® und Antarelix® von Asta Medica AG, Frankfurt/Main, Deutschland und Antide® von Ares-Sarono Int. AG, Lausanne, Schweiz.

Die genannten GnRH-Agonisten und GnRH-Antagonisten können in Dosierungen, zugelassen für andere Behandlungen, verabreicht werden. Ebenfalls können Dosierungen eingesetzt werden, die während Dosierungsfindungsstudien (Dose Finding Studies) für die Verwendung ähnlicher Stoffe (Substanzen, Medikamente) ermittelt wurden, wie beispielsweise Somatostatinanaloga bei Hypophysenadenom, Glioblastom oder Pankreasadenokarzinom oder für Phase II Studien mit GnRH Analoga (Agonisten oder Antagonisten) bei anderen Indikationen, z.B. Mammakarzinom, Prostatakarzinom oder Ovarielkarzinom.

Die GnRH-Agonisten oder GnRH-Antagonisten können mit einem Gonadotropin- bzw. LH-Hemmer z.B. Gossypol® (vgl. Flack et al., 1993. J. Endocrinol. Metab. Oral Gossypol in the Treatment of Metastatic Adrenal Cancer., 76, 1019-1024; Poso, H. et al., The Lancet 1980, 885) oder mit Melatonin oder einem Melatonin-Analogon (einem Agonisten oder Antagonisten) konjugiertern. (vgl. Lissoni et al., 1996, Increased Survival Time in Brain Glioblastomas by a Radioneuroendocrine Strategy with Radiotherapy plus Melatonin Compared to Radiotherapy Alone., Oncology, 53, 43-46).

Nachstehend folgt ein Beispiel eines bevorzugten Behandlungsprotokolls.

Erstmalig wurde die GnRH-Rezeptor-Konzentration in Zellmembranen von humanen Hirn- oder Nervensystemntumorzellen, d.h. die *in vitro* wirksamen GnRH-Rezeptoren auf der Membran, mittels Radio-Rezeptor-Assay bestimmt. Bei dem erfindungsgemäßen Verfahren wird die Bioaktivität bzw. spezifisch die aktiven GnRH-Rezeptoren bestimmt. Dazu wird radioaktiv markiertes Busereline, ein GnRH-Agonist, als Marker eingesetzt, der spezifisch an GnRH-Rezeptoren bindet. Anhand der radioaktiven Zerfälle des gebundenen Buserelinse läßt sich die GnRH-Rezeptor-Konzentration ermitteln. Dieser Nachweis wird bereits für andere Tumore wie Mammakarzinom und ähnliche eingesetzt. Das erfindungsgemäß verwendete Verfahren bestimmt die GnRH-Rezeptor-Konzentration auf Zellmembran-Extrakten von frischem humanem Tumorgewebe.

Während der peroperativen Entfernung des Tumorgewebes wird einerseits Gewebe für die pathologisch anatomische Begutachtung und andererseits Tumorgewebe für die GnRH-Rezeptorbestimmung gewonnen und verarbeitet, z.B. auf die hier beschriebene Art und Weise. Nach pathologisch anatomischer Begutachtung und Bestätigung der histologischen Diagnose eines Tumors, ausgehend vom Hirn und/oder vom Nervensystem und/oder von den Hirnhäuten und/oder vom Kaposi-Sarkom, kann eine Prognose für einen Therapieerfolg während einer Behandlung mit GnRH-Agonisten/Antagonisten anhand der vorhandenen GnRH-Rezeptor-Konzentration gegeben werden.

Bei einer Konzentration des GnRH-Rezeptors von mehr als 1000 amol/mg (=1 fmol/mg) Membranprotein wird der Patient als GnRH-Rezeptor positiv diagnostiziert. Das nicht GnRH-Rezeptor positiv sein, ist kein Ausschlußkriterium für die Behandlung, da es für die Behandlung mit GnRH-Agonisten/Antagonisten keine klinische Ausschlußkriterien gibt. Das GnRH-Rezeptor positiv sein eines Patienten wird beurteilt als prognostisch schnellere Rezidivtendenz als bei GnRH-Rezeptor negativ sein beim Verlauf des Tumorwachstums während klassischer Standardbehandlung, wobei der GnRH-Rezeptor als prognostischer Tumormarker fungiert. Auch wird das GnRH-Rezeptor positiv sein als besonders vorteilhaft für die Behandlung mit GnRH-Agonisten/Antagonisten beurteilt und gibt das GnRH-Rezeptor positiv oder negativ sein eine prognostische Aussage über den zu erwartenenen Therapieerfolg, so daß der GnRH-Rezeptor bei dieser Behandlung ein prognostischer Tumormarker ist. Die GnRH-Agonist/Antagonist-Behandlung wird sofort nach bekannter pathologisch anatomischer Begutachtung begonnen, z.B. postoperativ im Falle von Schnellschnitt pathologische Diagnostik.

Nach Bestimmung des Vorhandenseins von GnRH-Rezeptoren wird ein geigneter Ligand (GnRH-Agonist, GnRH-Antagonist oder Konjugate) selektiert und dem Patienten verabreicht, von dem der Tumor stammt, vorzugsweise nach diagnostischen Bildverfahren. Vgl. MTT Testliteratur: Hunter et al., 1993, Europ. J. Surg. Oncology, 242-249.

Die Behandlung wird fortgesetzt, solange noch keine komplette Remission eingetreten ist. Kriterien zur Beurteilung des Therapieeffektes sind: A. Tumorvolumen auf MRT-Bildern und/oder auf CAT-Scan-Bildern, B. Rezidivfreies Überleben, C. Gesamtüberleben nach Erstapplikation sowie D. Karnofsky- und Spitzer-Index. Die Dosierungen für die Verabreichung, die in jeder dem Fachmann bekannten geeigneten Form sein kann, sind vor- und nachstehend in dieser Patentanmeldung beschrieben.

Der genaue Wirkungsmechanismus von GnRH-Agonisten oder GnRH-Antagonisten auf Tumore ist unbekannt. Für die bereits bekannten Tumorarten mit aktiven GnRH-Rezeptoren wie z. B. Mammakarzinom, Prostatakarzinom und Ovarialkarzinom wird in der Literatur ein lokal regulatorisches autokrines-parakrines System vorgeschlagen; vgl. Irmer et al., 1995, Cancer Research 55, 817-822. In der Literatur sind für die genannten Tumore anti-proliferative Aktivitäten der GnRH-Agonisten oder GnRH-Antagonisten beschrieben worden, sowohl *in vitro* (Palyi et al., 1996, Cancer Delection and Prevention 20, 146-152; Irmer et al., 1995, Cancer Research 55, 817-822; Pati et al., 1995, Endocrinology 136, 75-84) als auch *in vivo* bzw. klinisch; vgl. Gonzalez-Barcena et al., 1994, The Prostate 24, 84-92; Jonat et al., 1995, European J. of Cancer 31A, 137-142; Emons und Schally, 1994, Human Reproduction Update 9, Nr. 7, 1364-1379. Diese anti-proliferative Aktivität geht dabei über den zu erwartenden anti-proliferativen Effekt der reversiblen "chemischen Kastration" durch GnRH-Agonisten hinaus.

Für Glioblastoma und Glioma kommt in gleicher Weise folgender Wirkungsmechanismus in Betracht. In der Literatur (Constam et al., 1992, J. Immunology 148, 1404-1410) wird die Produktion von Transforming Growth Factor β (TGF-β) durch Glioblastoma-Zellen beschrieben. Der Wachstumsfaktor TGF-β wird von Melcangi et al., 1995, Endocrinology 136, 679-686, als ein Produkt von Ratten-Gliazellen, d.h. normalen Nicht-Tumorzellen, beschrieben, der als Faktor *in vitro* die natürliche GnRH-Produktion in Hypothalamuszellen anregt. Postuliert wird, daß das örtlich (lokal) durch Glioblastoma produzierte und sezernierte GnRH eine stimulierende Wirkung auf das Wachstum des Tumors hat, was ebenfalls für TGF-β bekannt ist. Auch humane Glioblastomazellen bzw. Gliomazellen können zirkulierende immunsuppressive Substanzen sezernieren, vor allem TGF-β, und somit eine Beeinträchtigung zellulärer Immunreaktionen induzieren. Die Zunahme von TGF-β hat also wahrscheinlich außer einer GnRH-Produktion-stimuüerenden Funktion auch einen immunsuppressiven (abwehrhemmenden) Effekt auf die zelluläre Immunität des Patienten, wodurch das Tumorwachstum gefördert wird und die Tumorgröße zunimmt. Für Glioblastoma multiforme, Medulloblastom und malignes Melanom wurde dieses immunsuppressive Phänomen von TGF-β beschrieben; vgl. Stockhammer et al., 1995, J. of Neurosurgery 83, 672-681; Jennings et al., 1994, Hum. Pathol. 25, 464-475; Bizik et al., 1996, J. Cell Biochem. 62, 113-122; van Belle et al., 1996, Am. J. Pathol. 148, 1887-1894. Dieses autokrin-parakrin wachstumsregulierende System läßt sich auch umkehren, was eine Abnahme der Tumorgröße zur Folge hat. Diese Umkehrung (in der Endokrinologie "negatives Feedback" genannt) kann man im Prinzip mittels eines Überschusses an GnRH bewirken (kompetitive Inhibition). Dieser Effekt wird durch GnRH-Agonisten oder GnRH-Antagonisten an Stelle von GnRH noch verstärkt. Die Folge dieser Therapie ist eine Abnahme der TGF-β Produktion und eine daraus folgende Abnahme der Tumorgröße. Auch β-HCG hat eine immunsuppresive Rolle. Durch GnRH-Agonisten oder GnRH-Antagonisten wird erfindungsgemäß ebenfalls die LH-β- bzw. β-HCG-Produktion gehemmt. Auch wird bei GBM die EGF-Produktion gehemmt.

Für die Tumoren, mit GnRH-Rezeptoren vom Hirn neuroektodermalen Ursprungs und/oder den Hirnhäuten, die zur Indikationserfindung gehören wird verwiesen auf die World Health Organisation (WHO) Klassifikation der Tumoren des Zentralnervensystems, die 1990 ausgearbeitet wurde (Kleihues et al., 1993, Histological Typing of Tumours of the central nervous system, Springer Verlag, Berlin Heidelberg New York Tokyo). Außer den Tumoren, genannt in obengenannter WHO Klassifikation, gehören noch das maligne Melanom, und das Kaposi-Sarkom zur Indikationserfindung. Von der indikationserfindung ausgenommen sind das Hypophysenadenom, alle Metastasen außer Ewing Sarkom, Melanom und Kaposi-Sarkom, Lymphome und hämatopoetische Tumore. Die Keimzelltumoren, wie z.B. das Chorionkarzinom, ähneln den malignen Tumoren der Plazenta, von denen bekannt ist, daß diese GnRH-Rezeptoren haben. Das Kaposi-Sarkom, das multizentrisch im Körper vorkommt, besteht aus Zellen monoklonalen Ursprungs (Rabkin et al., 1996, The New England Journal of Medecine, 14, 988-993). Es hat spezifische Antigene gemeinsam mit dem Neurofibrom der Haut, einem Tumor, ausgehend vom Nervensystem (Rudolph, P. et al., 1997, Am. J. Surg. Pathol. (US), 21(7) 791-800).

Das Kaposi-Sarkom ähnelt hormonell den malignen Plazentatumoren und dem Meningiom, weil das Kaposi-Sarkom wie diese Tumoren β-HCG Rezeptoren besitzt und wie z.B. das Meningiom auf β-HCG Verabreichung antiproliferativ reagiert (Boyle-Wash et al., 1995, Effect of glycoprotein and protein hormones on human meningioma cell proliferation in vitro, Journal of Endocrinology, 145, 155-161; Albini et al., A., 1997, The beta-core Fragment of human chorionic gonadotropin inhibits growth of Kaposi sarcoma-derived cells and an new immortalized Kaposi sarcoma cell line, AIDS (US), 11 (6), 713-721); Gill et al., 1996, The effects of preparations of human chorionic gonadotropin on aids-related Kaposi Sarcoma, The New England Journal of Medicine, 335 (17), 1261-1269). Aufgrund der Analogie mit Meningioma hat Kaposi-Sarkom GnRH-Rezeptoren. Hierbei spielt der entdeckte autokrine Zusammenhang von GnRH als bekanntlich bei der Plazenta und bei Plazentatumoren, β-HCG freisetzendes Hormon eine Rolle (Lin et al., 1995, J. Clin. Endocrinol. Metab. 80: 580-585). Sowohl die obengenannten Tumoren in der WHO Klassifikation von Zentralnervensystemtumoren als auch das maligne Melanom mit β HCG Produktion und/oder mit β-HCG Rezeptoren haben GnRH-Rezeptoren. Das Ewing Sarkom gehört zu der Gruppe von primitiven neuroektodermalen Tumoren (PNET) und ist eine periphäre Form hiervon (Grier, H.E., 1997, The Ewing Family of Tumors. Ewing sarcoma and primitive neuroectodermal tumors. Pediatric Clin. North Am. (US), 44 (4), 991-1004).

Die Zirbeldrüse (Glandula pinealis) ist der Ursprung für die Produktion des Hormons Melatonin, ein GnRH-Rezeptor-Expression stimulierendes Hormon beim metastasiertem Prostatakarzinom im Falle von Resistenz während der GnRH-Agonist Behandlung (vgl. Lissoni et. al., 1997, European Urology 31, 178-181) und hat zusätzlich eine anti-angiogenetische Aktivität (Regelson, W., Pierpaoli, W., 1987, Cancer Invest., 5, 379-385). GnRH-Agonisten und GnRH-Antagonisten besitzen eine anti-mitotische bzw. anti-proliferative Aktivität durch Hemmung von Wachstumsfaktoren wie Epidermal Growth Factor (Motta et al., 1996, J. Steroid Biochem. Molec. Biol., 56, 107-11, 1996). Epidermal Growth Factor kommt auch als mitogen und somit als positiver Wachstumsfacktor, z.B. in Glioblastoma Multiforme vor (Rao et al., 1996, Peptides (US), 17, 179-181). Ein Melatonin-GnRH-Analog-Konjugat kombiniert somit sinnvoll eine anti-mitotische mit einer anti-angiogenetischen Aktivität auf Tumoren wie z.B. Glioblastoma und induziert die weitere Expression von GnRH-Rezeptoren in z.B. Glioblastoma multiforme, um eine Resistenz gegen GnRH-Agonist/Antagonist-Behandlung durch GnRH-Rezeptor-Depletion zu vermeiden.

Erfindungsgemäß werden erstmalig GnRH-Agonisten oder GnRH-Antagonisten zur Herstellung eines Arzneimittels bereitgestellt, um Tumore, mit-GnRH-Rezeptoren vom Hirn neuroektodermalen Ursprungs und/oder den Hirnhäuten und/oder Kaposi-Sarkoma und/oder malignes Melanom zu behandeln.

Die GnRH-Agonisten oder GnRH-Antagonisten und die konjugierten GnRH-Agonisten oder GnRH-Antagonisten dienen erfindungsgemäß zur Behandlung von Tumoren, mit-GnRH-Rezeptoren vom Hirn neuroektodermalen Ursprungs und/oder den Hirnhäuten, z.B. eines Glioblastoma multiforme. Die Arzneimittel der Erfindung können in jeder dem Fachmann bekannten Art und Weise hergestellt werden, insbesondere zur subkutanen, intramuskulären, intravenösen, intraspinalen bzw. subduralen oder intranasalen Applikation oder in Form eines Depot-Implantats. Die Arzneimittel der Erfindung können ebenfalls über ein subkutanes, ventrikuläres Cytostatikareservoir mit Verbindung zum Ventrikel verabreicht werden, wobei man durch die Haut hindurch das Reservoir mit Injektionen auffüllen kann. Die GnRH-Agonisten können in der gleichen Dosierung verabreicht werden, die z.B. für die Behandlung von Prostata-, Mammacarcinom oder Endometriose eingesetzt werden; vgl. z.B. Rote Liste, 1997, Abschnitt 50, Teil 3. Hypothalamushormone, 50038 bis 50056, Herausgeber ROTE LISTE® Service GmbH, Frankfurt/Main, auf die die hier ausdrücklich Bezug genommen wird: vgl. Annex A. Die minimale Dosierung entspricht der in der Roten Liste für die jeweiligen GnRH-Agonisten angegebenen Dosis. Bei z.B. der intraspinalen oder subkutanen, ventrikulären Verabreichung über ein Cytostatikareservoir kann die minimale Dosierung niedriger sein als in der Roten Liste für die jeweiligen GnRH-Agonisten angegeben ist. Die maximal Dosierung entspricht dem $LID_{50}$-Wert für die entsprechenden CmRH-Agonisten. Die Dosierung kann gegebenenfalls nach einem neurologisch erhaltenen Befund der GnRH-Rezeptor-Konzentration erhöht oder erniedrigt werden. Die Häufigkeit der Applikation bzw. die Tagesdosis kann ebenfalls der Roten Liste entnommen werden. Die Arzneimittel werden vorzugsweise bis zur kompletten Remission (Rückbildung) des Tumors, die neuroradiologisch und klinisch festgestellt werden kann, verabreicht.

Zur subkutanen Verabreichung können z.B. Carcinil®, Decapeptyl® 0,5 mg/0,1 mg oder Uno-Enantone eingesetzt werden. Als Depot-Implantate können z.B. Profact®-Depot, Zoladex® oder Enantone Monatsdepot verabreicht werden. Zur intramuskulären Verabreichung können z.B. Decapeptyl®-Depot, Decapeptyl®-Gyn oder Enantone-Gyn eingesetzt werden. Zur intranasalen Verabreichung können z.B. Profact®-Nasal, Suprecur®-Nasal oder Synarela®-Nasal eingesetzt werden. Zur intravenösen Verabreichung bzw. zur intranasalen Verabreichung kann z.B. Profact pro injectione / -nasal in den Dosierungen verabreicht werden, die von Klijn, J.G. und De Jong, F.H. angegeben sind in Klijn, J.G. und De Jong F.H., 1982, The Lancet, 1213-1216. Die GnRH-Antagonisten können in der Dosierung verabreicht werden, die z.B. für Cetrolix® in Gonzalez-Barcena et al., 1994, The Prostate 24, 84-92, angegeben ist oder minimal in der Dosierung verabreicht werden die z.B. für Antide® angegeben ist in Fattinger et al., 1996, Am. J. Physiol. 271 (Endocrinol. Metab. 34): E775-E787).

Die folgenden Beispiele erläutern die Erfindung und sind nicht als beschränkend aufzufassen.

Beispiel 1: GnRH-Rezeptor-Konzentrationsbestimmung

Als Beispiel einer GnRH-Rezeptor-Konzentrationsbestimmung auf Zellmembranextrakten von Zellinien und/oder von Zellkulturen wird der Decapeptyl®-Radio-Rezeptor-Assay mit Membranen durchgeführt (wie beschriebem in Emons, G. et al., 1993, Cancer Research 53, 5439-5446). Auf eine humane Zellinie, wie die humane Glioblastoma-Zellinie U-87 MG oder U-373MG (Pinski et al., 1994, Cancer Research 54, 5895-5901) werden die GnRH-Rezeptoren nach dieser Vorschrift bestimmt. Hierbei werden sowohl die Low Affinity/High Capacity als auch die High Affinity/Low Capacity-GnRH-Rezeptor-Bindungsstellen bestimmt. Es werden ähnliche Resultate erhalten, wie die die bei Emons G. et al., *supra,* für die Zellinien EFO-21 und EFO-27 beschrieben sind.

Als weiteres Beispiel einer GnRH-Rezeptor-Konzentrationsbestimmung auf Zellmembranextrakten von Zellinien und/oder Zellkulturen wird der LHRH-Radio-Rezeptor-Assay mit markiertem Triptorelin (Emons G. et al., *supra*) auf einer Kaposi-Sarkoma-Zellinie, so wie die bekannte Zellinie KSY-1 oder KS-SLK (Parkash et al., 1996, New England Journal of Medicine 335, 17, 1261-1269) und auf eine humane maligne Melanoma-Zellinie, wie die bekannten Zellinien MV3 und BLM (Goldbrunner, R.H. et al., 1996, Anticancer Research 16 (6B), 3679-3687) durchgeführt mit ähnlichen Resultaten der GnRH-Rezeptorbestimmungen wie beschrieben in Emons, G. et al., *supra,* für die Zellinien EFO-21 und EFO-27.

Beispiel 2: GnRH-Rezeptor mRNA-Bestimmung, mittels RT-PCR

Als Beispiel einer GnRH-Rezeptor messenger RNA Bestimmung mittels RT-PCR wird z.B. RNA aus der Glioblastoma Zellinie U-87 MG oder U-373MG in einer ersten Reaktion in cDNA umgeschrieben. In einer weiteren Reaktion wird z.B. das 884 bp lange Fragment des hypophysen GnRH-Rezeptors (Kakar, S. et al., Biochem. Biophys. Res. Comm., 1992, 289-295) oder z.B. des Plazenta-GnRH-Rezeptors (Leung, P.C.K., Biological Signals 1996, 5, 63-69) oder des Plazenta-GnRH-Rezeptor-Gens (Lin, L. et al., J. Clinical Endocrinol. Metabolism 1995, Bd. 80, Nr. 2, 581-584) mit spezifischen Primern in einer Reverse-Transkriptase-Polymerase-Kettenreaktion amplifiziert. Hierbei dient die cDNA eines bekannten GnRH-Rezeptor positiven Zellinie als positive Kontrolle. Die Reaktionsprodukte werden dann auf einem Polyacrylamid (PAA)-Gel sichtbar gemacht. Auf dem PAA- Gel sieht man in Spur I den Fragmentgrößen-Marker, in Spur 2 eine deutliche Bande des 884bp langen GnRH-Rezeptor PCR Produktes bei der MCF 7 Positivkontrolle und in der Spur der Glioblastoma Zellinie ebenfalls ein 884bp Produkt Signal oder andere GnRH Rezeptor-Splice-Variant-(Fragment) Signale. Diese mRNA Bestimmung wird ähnlich wie andere GnRH-Rezeptor mRNA Bestimmungen durchgeführt, siehe z.B. Irmer et al., 1995, Cancer Research, 55, 817-822.

Beispiel 3: Therapeutische *in vitro*-Studie

Proliferations-Assay auf Zellkulturen.

Eine humane Zellinie wie die bekannte humane Glioblastoma Zellinien U-87MG oder U-373MG (Pinski et al., *supra*) oder eine humane Zellinie wie die bekannte Kaposi-Sarkoma Zellinien KSY-1 oder KS-SLK (Parkash et al., 1996, New England Journal of Medecine, 335, 17, 1261-1269) oder eine humane Zellinie wie die bekannte humane maligne Melanoma Zellinie MV 3 oder BLM (Goldbrunner, R.H. et al., 1996, Anticancer Research, 16 (6B), 3679-87) oder eine humane Medulloblastoma Zellinie wie die bekannte Zellinie Daoy oder D283 MED (Stockhammer et al., 1995, J. Neurosurgery, 83, 672-681) oder humane Meningioma Zellkulturen (Boyle-Wash, E. et al., 1995, Journal of Endocrinology 145, 155-161) wird in Kultur gebracht, wie von obengenannten Autoren für die obengenannten Zellinien beschrieben und dann wie beschrieben von Emons, G. et al., 1993, supra und Irmer, G. 1995, supra behandelt mit den dort angegebenen Konzentrationen von GnRH-Agonist-Triptorelin, GnRH-Antagonist SB-75 (Cetrorelix®) oder GnRH-Antagonist Ramorelix®. Ähnliche Resultate wie von Emons et al., Cancer Research 53, 1993, 539-544 und Irmer, G. et al., supra beschrieben wurden, erhalten.

Die obengenannten Zellinien wurden separat davon ebenfalls behandelt mit einem GnRH-Agonisten, entweder Goserelin (Zoladex®, Buserelin oder Leuprorelin, oder mit einem GnRH-Antagonisten, wie Antide® oder Antarelix®. Ähnliche anti-proliferative Effekte wurden beobachtet, wie von Pinski et al. oder Irmer et al., *supra,* beschrieben.

Separat davon werden solche Zellinien ebenfalls behandelt mit jeweils einem der GnRH-Antagonisten Cetrorelix®, Antarelix®, Antide® und Ramorelix® oder mit einem GnRH-Antagonisten wie beschrieben in US-Patent 5,480,969, US-Patent 5,198,533 oder UK-Patent GB 2 246782 B und zwar auf ähnliche Weise wie bei Emons et al., *supra,* für SB 75 (Cetroretix®) angegeben. Ein ähnlicher anti-proliferativer Effekt tritt auf.

Die obengenannten Zellinien werden separat davon ebenfalls behandelt mit monoklonalen Antikörpern gegen ein GnRH-Rezeptor Antigen, wie beschrieben von KARANDE, A.A. et al., 1995, Mol. Cell. Endocrinol. 114 (1-2), p. 51-56. Ein ähnlicher anti-proliferative Effekt wird bei den obengenannten Zellinien beobachtet wie von ACKERMANN, R. C. et al., 1994, Cancer Letters, 81. 177-184 für die Zellinie OVCAR-3 beschrieben worden ist.

Beispiel 4: *In vivo*-Studie im Xenotransplantations-Modell

Eine *in vivo*-Studie auf Nacktmäusen

Der Effekt der Behandlung von mit Tumoren implantierten Nacktmäusen (Pinski et al., *supra*) mit jeweils einem der GnRH-Agonisten Buserelin, Triprorelin, Goserelin und Leuprorelin und mit jeweils einem der GnRH-Antagonisten Cetrorelix® (SB-75), Antarelix®, Antide® und Ramorelix® auf das Wachstum von malignen Gliomen U-87 MG und U-373MG wird von uns nachgewiesen mit Tagesdosierungen und Kontrollen bei Nacktmäusen, wie für den Wirkungsnachweis für ähnliche Peptide beschrieben wurde in Pinski et al., *supra.* Ähnliche wachstumshemmende Effekte konnten bei den genannten Tumoren durch Behandlung mit den von uns hier genannten GnRH-Agonisten und GnRH-Antagonisten beobachtet werden.

Beispiel 5: Phase I-Studie

Patienten mit Rezidiv eines nicht rezesierbaren Glioblastoma multiforme bei Zustand nach mikrochirurgischer Resektion und/oder nach externer konventioneller Strahlentherapie und/oder Brachytherapie oder Patienten mit einem diffus intraaxial wachsenden Hirntumor, multifokale Ausdehnung der Geschwulst bzw. Vorliegen einer Gliomatosis Cerebri, ein Tumorvolumen von mehr als 65 ml oder ein minimaler Tumordurchmesser von mehr als 5 cm werden behandelt mit GnRH-Agonist Buserelin in der intravenöse Verwendung wie beschrieben von Klijn, J.G.M.et al., 1982, The Lancet, Mai 19, 12143-1214. und in ebenfalls mit der dort beschriebenen intranasalen Applikation als Dauermedikation. Als Behandlungseffekt tritt eine Tumorvolumenreduzierung auf MRT bzw. CT - Bildern auf. Ein rezidivfreies Überleben, das länger ist, als für die Tamoxifen-Behandlungsmethode von Glioma (Pollack et al., 1995, Pediatr. Neurosurgery 22, 281-288) beschrieben ist, wurde beobachtet.

Beispiel 6: Phase I-Studie

2 Patienten mit inoperabelen, stereotaktisch bestätigtem Glioblastoma multiforme werden nach konventionellen Strahlentherapie behandelt mit Zoladex® in der Dosierung und Verabreichungsform unter Dauermedikation, welche für das metastasierte Mammakarzinom in der Roten Liste angegeben wird. Auf den MRT Kontrollen zeigt sich eine signifikante Tumorvolumenreduktion.

Beispiel 7: Phase II-Studie

Patienten mit histologisch bestätigtem Glioblastoma multiforme nach einer ersten Operation werden (randomized controlled) behandelt mit Zoladex®, wie beschrieben in Jonat et al., 1995, European J. Cancer, 137-142. Nach der Strahlentherapie werden sie unterteilt in 2 Gruppen. Eine Gruppe wird mit Zoladex® und eine Gruppe ohne Zoladex® (oder mit Cetrorelix® und ohne Cetrorelix® oder mit Antide® und ohne Antide® oder mit Decapeptyl® oder ohne Decapeptyl® usw.) behandelt. Die Effekte sind ähnlich wie beim metastasiertem perimenopausalem Mammakarzinom. Der Prozentsatz mit objektivem signifikanten Therapieeffekt wird beurteilt unter den Kriterien Tumorvolumen, rezidivfreies Überleben, Gesamtüberleben nach Erstapplikation und Kamofsky- und Spitzer Index bei klinisch neurologischer Untersuchung und unter Berücksichtigung der weiteren Untersuchungskriterien (Sposto, R. et al., 1989, J. Neurooncology, 7, 165-177, und Kirby, S. et al., 1995, J. Natl. Cancer Institute, 87, 1884-1888, 1995). Im MRT und/oder CAT Scan wird eine signifikant größere Tumorvolumenreduzierung bzw. signifikant längeres rezidivfreies Überleben und ein signifikant längeres Gesamtüberleben nach Erstapplikation festgestellt als bei der nicht mit Zoladex® behandelte Kontrollgruppe.

Mit einer dem Fachmann bekannten Methode von Gentherapie werden Retroviren und Antisense-GnRH-Rezeptor-Vektoren stabil transfektiert in Glioma-Zellinien und es tritt ein anti-proliferativer Effekt auf.

Beispiel 8: Die Sammlung von Gliomagewebe

Während Hirntumoroperationen (peroperativ) wurde frisches, humanes Tumorgewebe trocken in einem sterilen Schälchen ohne Zufügung von Medium gesammelt und sofort in ein steriles Standard-Plastikröhrchen überführt. Das Röhrchen wurde luftdicht verschlossen und nach etwa 15 Minuten in einem Dewar-Gefäß (Union Carbide Cryogenic Equipment 35HC, Serien-Nr. 103-139-T5), das flüssigen Stickstoff enthielt, schockgefroren. Die Gewebeproben wurden etwa 2 Monate in flüssigem Stickstoff bis zur GnRH-Rezeptorbestimmung aufbewahrt.

Beispiel 9: Gewebeaufarbeitung

Die gefrorenen Gewebeproben wurden von Blut- und Fettresten befreit und mit einem Skalpell in ca. 2 x 2 x 2 mm große Stücke zerschnitten. Die Gewebeproben wurden 1 Minute bei maximaler Leistung in einem Dismembrator II (B. Braun, Melsungen) homogenisiert. Das homogenisierte Gewebe wurde in 1000 µl kaltem Puffer 1 (10 mM Tris (hydroxymethyl)-aminomethan, pH 7,4, 4°C) aufgenommen und möglichst homogen durchmischt. In einem ersten Zentrifugationsschritt (800 x g, 10 Minuten, 4°C) wurde die Probe von größeren Gewebetrümmern getrennt. Der Überstand wurde erneut zentrifugiert (10.000 x g, 45 Minuten, 4°C). Der Überstand des zweiten Zentrifugationsschritts wurde verworfen und das Pellet, das die Membranfraktion enthielt, in 1000 µl kaltem Puffer 1 aufgenommen und mit einem Polytron-Homogenisator dreimal je 4 Sekunden homogenisiert, um eine möglichst homogene Membransuspension zu erhalten. Zu dieser Membranfraktion wurden 1000 µl kalter Puffer 1 zugesetzt. Diese Suspension wurde zur GnRH-Rezeptorbestimmung im Radio-Rezeptor-Assay eingesetzt.

Beispiel 10: Proteinkonzentrationsbestimmung

Das Bio-Rad-Reagenz wurde im Verhältnis 1:5 mit destilliertem Wasser verdünnt. 3,5 ml dieses Reagenz wurde mit 50 µl der präparierten Membranfraktion gemischt und 10 Minuten inkubiert. Die photometrische Messung der Proteinkonzentration erfolgte als Zweifachbestimmung bei lambda ist gleich 595 nm in bekannter Weise. Als Proteinstandard diente ein Humanalbumin-Proteinstandard, mit dem in entsprechender Weise die Messungen ausgeführt werden.

Beispiel 11: Der Radio-Rezeptor-Assay

Die Bestimmung der GnRH-Rezeptorkonzentration erfolgte in der wie vorstehend beschriebenen präparierten Membranfraktion des Gewebes. Der Radio-Rezeptor-Assay umfaßte zwei verschiedene Probenansätze, die jeweils vierfach bestimmt wurden: (a) Probenansätze mit der präparierten Membranfraktion und (b) Kontrollansätze.

a) zu 100 µl Membranfraktion wurden 300 µl Puffer 2 (10 mM Tris(hydroxymethyl)-aminomethan, pH 7,4, 0,1% Rinderserumalbumin) und 100 µl Tracer ($^{125}$J-Buserelin, 80.000 cpm/100µl) zugesetzt.

b) für die Kontrollen wurden 250 µl Puffer 2, 100 µl Tracer, 100 µl Membranfraktion und 50 µl GnRH-Analogon ($10^{-5}$ M Buserelin) vermischt.

Die einzelnen Proben wurden dann gut durchmischt und anschließend 90 Minuten bei 4°C inkubiert. Der Radio-Rezeptor-Assay wurde durch Zugabe von 500 µl Rindergammaglobulinlösung (0,1 % Pindergammaglobulin, 0,15 M NaCl) gestoppt. Danach wurden 1000 µl einer 25% PEG-6000, 0,15 M NaCl-Lösung zugesetzt.

Die Proben wurden nochmals homogen durchmischt und 20 Minuten bei 4°C inkubiert. Die Abtrennung der PEG-Hormon-Rezeptor-Komplexe erfolgte mit einem Zentrifugationsschritt (1.600 x g, 30 Minuten, 4°C), bei dem die Komplexe aufgrund ihrer höheren Masse das Pellet bilden. Der Überstand wurde vorsichtig mit einer Pasteur-Pipette abgesaugt. Die Anzahl der Zerfälle pro Minute, die als Basis für die Berechnung des GnRH-Rezeptor-Gehalts dient, wurde anschließend mit einem Gamma-Counter (Berthold) gemessen.

Beispiel 12: Überprüfung des Radio-Rezeptor-Assays

Gewöhnlich wurden mehrere Gewebeproben in einem Versuchsansatz untersucht. Zum Ausschluß eines systematischen Fehlers bei einem negativen Ergebnis aller Proben in einem Assay, wurde bei jedem Assay eine Standardprobe aus Rinder-Hypophysengewebe parallel zu den Tumorgewebeproben untersucht. Der Nachweis von GnRH-Rezeptoren in Rinder-Hypophysengeweben diente somit als Positivkontrolle. Das Hypophysengewebe wurde wie die Tumorgewebeproben aufgearbeitet und die Membranfraktion in der gleichen Weise gereinigt.

Beispiel 13: Berechnung des GnRH-Rezentorgehalts

Die Berechnung des GnRH-Rezeptorgehalts (fmol/mg Membranprotein) erfolgte aufgrund der Anzahl der Zerfälle pro Minute (cpm), der spezifischen Bindung, der eingesetzten . Proteinmenge und der spezifischen Aktivität des radioaktiv markierten Liganden.

Die spezifische Bindung ($B_{spez}$) ergibt sich aus der Differenz des Mittelwerts der Vierfachbestimmung der Gesamtbindung ($B_o$) und des Mittelwerts der Vierfachbestimmung der unspezifischen Bindung (NSB).

Die eingesetzte Proteinmenge wird, wie vorstehend bei Ziffer 3 dargestellt, photometrisch bestimmt.

Daten des Analogons $^{125}$J-Buserelin:

| | |
|---|---|
| MG: | 1253 g/mol |
| Spezifische Aktivität: | 1470 mCi/mg |
| Aktivität der $^{125}$J-Buseretin-Lösung: | 20 µCi/ml |

- 1470 mCi/mg $^{125}$J-Buserelin = 54,4 x $10^9$ Bq/mg
- 1 ml $^{125}$J-Buserelin-Lösung beinhalten 13,61 x $10^{-9}$ g $^{125}$J-Buserelin mit 7,4 x $10^6$ Bq
- 13,61 x $10^{-9}$ g/ml $^{125}$J-Buserelin = 10,9 x $10^{-12}$ Mol $^{125}$J-Buserelin. 54,4 x $10^9$ Bq = 44,4 x $10^7$ cpm
- 10,90 x $10^{-12}$ Mol $^{125}$J-Buserelin = 44,4 x $10^7$ cpm
- 1000 cpm entsprechen 0,247 x $10^{-15}$ Mol $^{125}$J-Buserelin

Zur Berechnung der GnRH-Rezeptorkonzentration (fmol/mg Membranprotein) aus den gemessenen cpm-Werten, muß nun noch die eingesetzte Proteinmenge und der Zerfallfaktor berücksichtigt werden. Die Formel zur Berechnung des GnRH-Rezeptorgehalts lautet somit:

$$\frac{0{,}247 \times 10^{-15}\ \text{Mol}\ ^{125}\text{J-Buserelin}}{\text{Zerfallsfaktor} \times \text{Proteinmenge}} = 1000\ \text{cpm}$$

## Tabelle II:

Bestimmung der GnRH-Rezeptorkonzentration

Aufgeführt sind Ergebnisse der GnRH-Rezeptor-Bestimmung mit dem erfindungsgemäßen Radio-Rezeptor-Assay von Gewebeproben verschiedener Patienten.

| Histologieproben | ER fmol/mg/Prot | PgR fmol/mg/Prot | GnRH-Rez atomol/mg/Prot | Befund |
|---|---|---|---|---|
| | 10 | 20 | 1000 | negativ |
| | 10-20 | 20-30 | 1000-3000 | schwach positiv |
| | 20 | 30 | 3000-5000 | positiv |
| | 50 | 100 | 5000 | stark positiv |
| Chordom | 1 | 1 | 708 | |
| GBM | 1 | 2 | 2478 | |
| GBM | 1 | 1 | 895 | |
| GBM | 1 | 1 | 1111 | |
| Gliom G II | 1 | 1 | 3635 | |
| Meningeom | 1 | 74 | 1 | |
| Adenocarcinom | 1 | 1 | 1 | |
| GBM | 1 | 1 | 7357 | |
| Fibrillär Astrozytom G II | 1 | 1 | 1 | |
| Meningeom | 1 | 177 | 7444 | |
| Meningeom | 1 | 550 | 1588 | |
| GBM | 1 | 1 | 4466 | |

ER=Östrogen-Rezeptor, PgR=Progesteron-Rezeptor, GnRH-Rez=GnRH-Rezeptor

zusätzliche Werte:

| | | | | |
|---|---|---|---|---|
| -Chordom | 1 | 1 | 1117 | schwach positiv |
| -Meningiom intraspinal | 3 | 7 | 1640 | schwach positiv |
| -Hirn Metastase eines Platteneptithelzell karzinoms der Lunge | 1 | 1 | 200 | negativ |
| -Normales Hirngewebe | 4 | 1 | 460 | negativ |

Beispiel 14: Proliferations assay mit der humanen maligne Melanoma Zellinie MV3

Die humane Melanoma Zellinie MV3 wurde in Kultur gebracht (in Lanzeitkultur in RPMI Medium (Gibbco Co.) mit 1% Penstrep und 10% heat inactivated Fetal Calf Serum). Der Proliferationsassay wurde durchgeführt mit 6 x $10^2$ Zellen pro Well in 96-Well-Platten. Die Zellen wurden zuerst von der Kulturflasche mit einer 0,02 mM Lösung abgelöst und dann gewaschen mit PBS-Standardlösung. Nach Zentrifugieren für 10 Minuten (1200 g) wurde der Überstand abgegossen und das Pellet in 1 ml Medium aufgenommen. Dazu nahm man 1 Aliquot von 20 microl der Zellen, welches mit Trypanblau verdünnt wurde in einer 1 zu 20 Verdünnung. Das Trypanblau färbt die nekrotischen Zellen an. Dann erfolgte die Zählung in der Neubauer Zählkammer. Dann erfolgte eine Auswertung, indem man anfangend mit Tag 0 täglich 4 Werte bestimmt und für die Zellzahl in einem ml die Zellzahl-Mittelwerte x $10^4$ x Verdünnungsfaktor 20 multipliziert. Es wurde während 5 Tage 4 x täglich gemessen mit einem Biomec spectrofotometer.

Die Methode wie die Proliferation der Tumorzellen gemessen wird, ist beschrieben in Lü, H.Q. et al., 1996, Journal of Cancer Research and Clinical Oncology, 122, 335-342.

Die Zellinie wurde behandelt mit (Gly-OH10) - LHRH, das LHRH Hormon (Figur 3), (Sigma Chemical Co., No. L8008) oder TRIPTORELIN, ein LHRH Agonist (Figur 2), (Sigma Chemical Co., No. L9761) oder ANTIDE, ein LHRH Antagonist (Figur 1), (Sigma Chemical Co., No. A8802).

Bei den Konzentrationen $10^{-4}$ M, $10^{-5}$ M und $10^{-6}$ und mit dem Medium als negativer Kontrolle wurden ab Tag 4 folgende Ergebnisse erhalten:

Für ANTIDE (GnRH Antagonisten) ist in Fig. 1 eine deutliche Proliferationshemmung zu sehen in den hohen Konzentrationen $10^{-4}$ M und $10^{-5}$ von 15% bzw. 35% (ähnlich wie beschrieben von EMONS et al., 1993, supra aber später einsetzend als beim einer der dortigen Ovariumkarzinotnzellinien, wo ab Tag 1 ein antiproliferativer Effekt der Antagonisten bei einer der zwei Zellinien auftrat). Bei der Konzentration $10^{-6}$ wurde keine Proliferationshemmung gesehen, sondern eine Stimulierung des Wachstums von 40%. Dieser pardoxale IN VITRO Effekt von GnRH ANTAGONISTEN ist ähnlich wie bei LIMONTA et al., 1993, J. Clin. Endocrinol. Metab., 76, 839-845 beschrieben für Prostatakarzinom mit GnRH Rezeptoren. Ein ähnlicher in vitro Effekt für relativ niedrige Konzentrationen ist ebenfalls bekannt für Tamoxifen bei der MCF-7 Mammakarzinom Zellinie (Zänker, K. et al., 1995).

Für TRIPTORELIN (GnRH Agonist) (siehe Figur 2) wurde ab Tag 4 eine Proliferationshemmung von 15% festgestellt bei den genannten Konzentrationen. Bei Emons et al., 1993, supra war dies bereits ab Tag 1 für beide Ovariumkarzinomzellinien bei $10^{-5}$ M Triptorelin Behandlung zu beobachten und am Tag 6 wurde 40% Hemmung beobachtet.

Diese Befunde beweisen das Vorhandensein eines direkten antiproliferativen Effektes von Antide und Triptorelin auf maligne Melanoma. Ebenfalls wird bewiesen, daß GnRH Rezeptoren auf der humanen malignen Melanoma Zellinie MV 3 vorliegen, da eine Nicht-Ligand Bindung an den Tumorzellen ausgeschlossen ist.

Die Kurven der Figuren 1-3 beweisen, daß das maligne Melanoma MV 3 ein LHRH Hormonabhängiger Tumor ist.

Das LHRH-Hormon funktioniert somit in vitro als positiver Wachstumsfaktor. Die Funktion von autokrin-produziertem LHRH-Hormon wird gehemmt von Antide und Triptorelin.

## Patentansprüche

1. *Ex vivo* Verfahren zur Erkennung und Bestimmung von GnRH-Rezeptoren auf Tumorzellen vom Hirn neuroektodermalen Ursprungs und/oder Hirnhäuten und/oder vom malignen Melanom und/oder vom Kaposi Sarkom, umfassend das Kontaktieren genannter Zellen mit einem Ligand für einen GnRH-Rezeptor, und Bestimmen, ob eine Bindung stattgefunden hat.

2. Verfahren nach Anspruch 1, **dadurch gekennzeichnet, dass** der Ligand ein Antikörper ist.

3. Verfahren nach Anspruch 1 oder 2, **dadurch gekennzeichnet, dass** der genannte Ligand markiert ist.

4. Verfahren nach Anspruch 1, **dadurch gekennzeichnet, dass** der gebundene Ligand bestimmt wird mit markiertem Anti-Ligand, vorzugsweise einem Antikörper.

5. *Ex vivo* Verfahren zur Erkennung und Bestimmung von GnRH-Rezeptoren auf entarteten Zellen eines Tumors vom Hirn neurocktodermalen Ursprungs und/oder den Hirnhäuten, umfassend:

   a) Homogenisieren von peroperativ gesammeltem Tumorgewebe,
   b) Abtrennen der Membranfraktion,
   c) Bestimmen der Proteinkonzentration in der Membranfraktion von b) und
   d) Bestimmen der GnRH-Rezeptorkonzentration in der Membranfraktion von b) zur Diagnose der genannten Tumoren.

6. Verwendung von GnRH-Agonisten oder GnRH-Antagonisten zur Herstellung eines Arzneimittels zur Behandlung eines Tumors mit GnRH-Rezeptoren vom Hirn neuroektodermalen Ursprungs und/oder den Hirnhäuten, und/oder zur Behandlung eines malignen Melanoms und/oder zur Behandlung eines Kaposi-Sarkoms.

7. Verwendung nach Anspruch 6, wobei die GnRH-Agonisten oder GnRH-Antagonisten mit einer zytotoxischen Substanz konjugiert sind.

## Claims

1. An *ex vivo* method for the detection and determination of GnRH receptors on tumor cells from the brain of neuroectodermal origin and/or meninges and/or of malignant melanoma and/or of Kaposi sarcoma, comprising contacting of said cells with a ligand for a GnRH receptor and determining whether a binding occurred.

2. Method according to claim 1, **characterized in that** the ligand is an antibody.

3. Method according to claim 1 or 2, **characterized in that** said ligand is marked.

4. Method according to claim 1, **characterized in that** the bound ligand is determined by a marked anti-ligand, preferably an antibody.

5. *Ex vivo* method for the detection and determination of GnRH receptors on degenerated cells of a tumor from the brain of neuroectodermal origin and/or the meninges, comprising:

   a) homogenizing of peroperatively collected tumor tissue,
   b) separation of the membrane fraction,
   c) determination of the protein concentration within the membrane fraction of b), and
   d) determination of the GnRH receptor concentration within the membrane fraction of b) for diagnosing said tumors.

6. Use of a GnRH agonist or a GnRH antagonist for the preparation of a medicament for the treatment of a tumor from the brain of neuroectodermal origin with GnRH receptors and/or of the meninges and/or for the treatment of malignant melanoma and/or for the treatment of Kaposi sarcoma.

7. Use according to claim 6, wherein the GnRH agonists or GnRH antagonists are conjugated to a cytotoxic substance.

## Revendications

1. Procédé *ex vivo* d'identification et de détermination de récepteurs GnRH sur des cellules tumorales du cerveau d'origine neuroectodermique et/ou méningées et/ou de mélanome malin et/ou de sarcome de Kaposi, comprenant la mise en contact des cellules citées avec un ligand d'un récepteur GnRH, et la détermination de la réalisation ou non d'une liaison.

2. Procédé selon la revendication 1, **caractérisé en ce que** le ligand est un anticorps.

3. Procédé selon la revendication 1 ou 2, **caractérisé en ce que** le ligand cité est marqué.

4. Procédé selon la revendication 1, **caractérisé en ce que** le ligand lié est déterminé avec un anti-ligand marqué, de préférence un anticorps.

5. Procédé *ex vivo* d'identification et de détermination de récepteurs GnRH sur des cellules dégénérées d'une tumeur du cerveau d'origine neuroectodermique et/ou méningées, comprenant :

   a) l'homogénéisation de tissus tumoraux recueillis avant opération,
   b) la séparation de la fraction membranaire,
   c) la détermination de la concentration en protéines dans la fraction membranaire de b)
   et
   d) la détermination de la concentration en récepteur GnRH dans la fraction membranaire de b) pour le diagnostic des tumeurs citées.

6. Utilisation d'agonistes GnRH ou d'antagonistes GnRH pour la production d'un médicament pour le traitement d'une tumeur du cerveau avec des récepteurs GnRH d'origine neuroectodermique et/ou méningée et/ou pour le traitement du mélanome malin et/ou pour le traitement du sarcome de Kaposi.

7. Utilisation selon la revendication 6, les agonistes GnRH ou les antagonistes GnRH étant conjugués avec une substance cytotoxique.

Figur 1 Antide

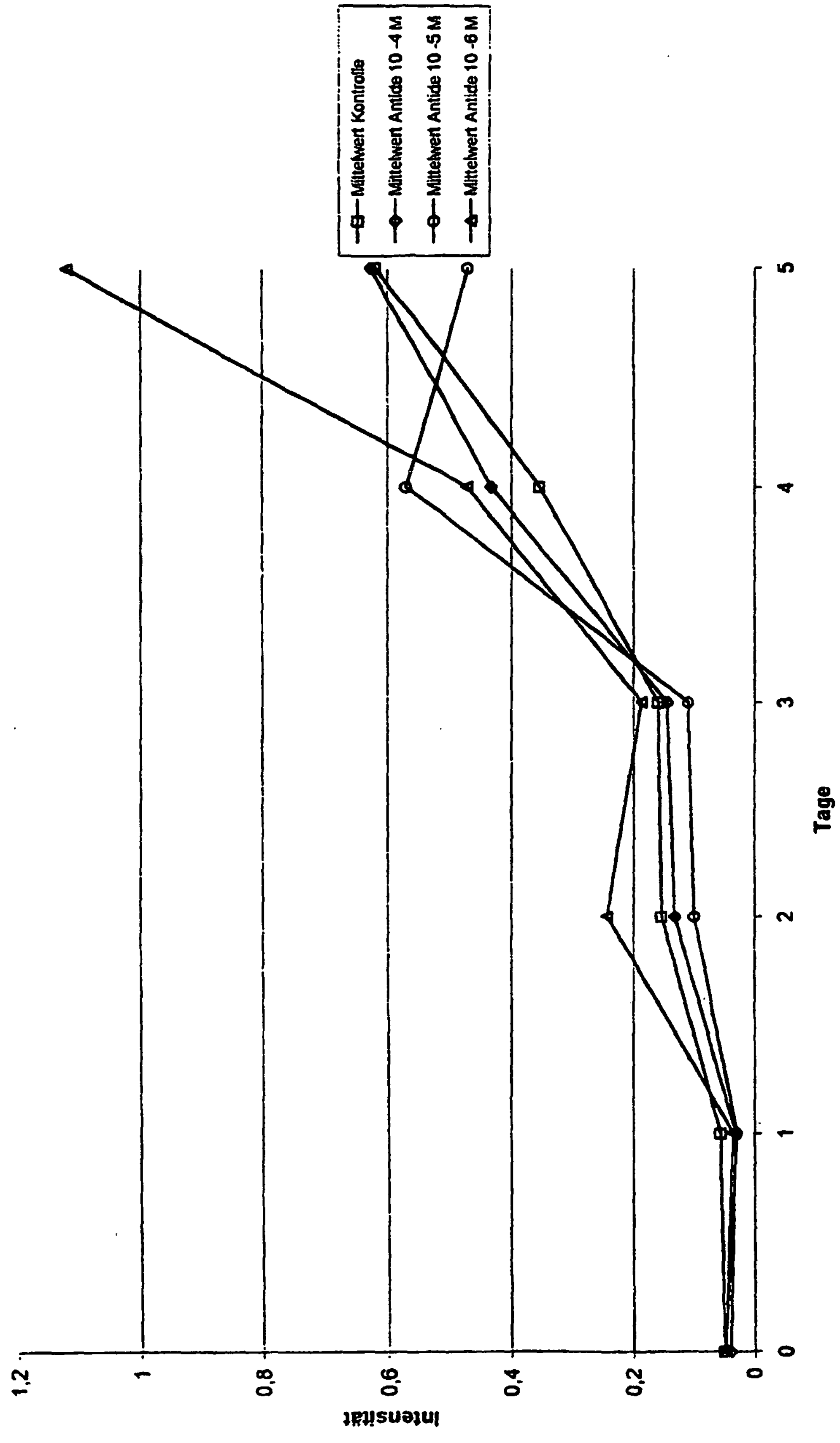

Figur 2 triptorelin

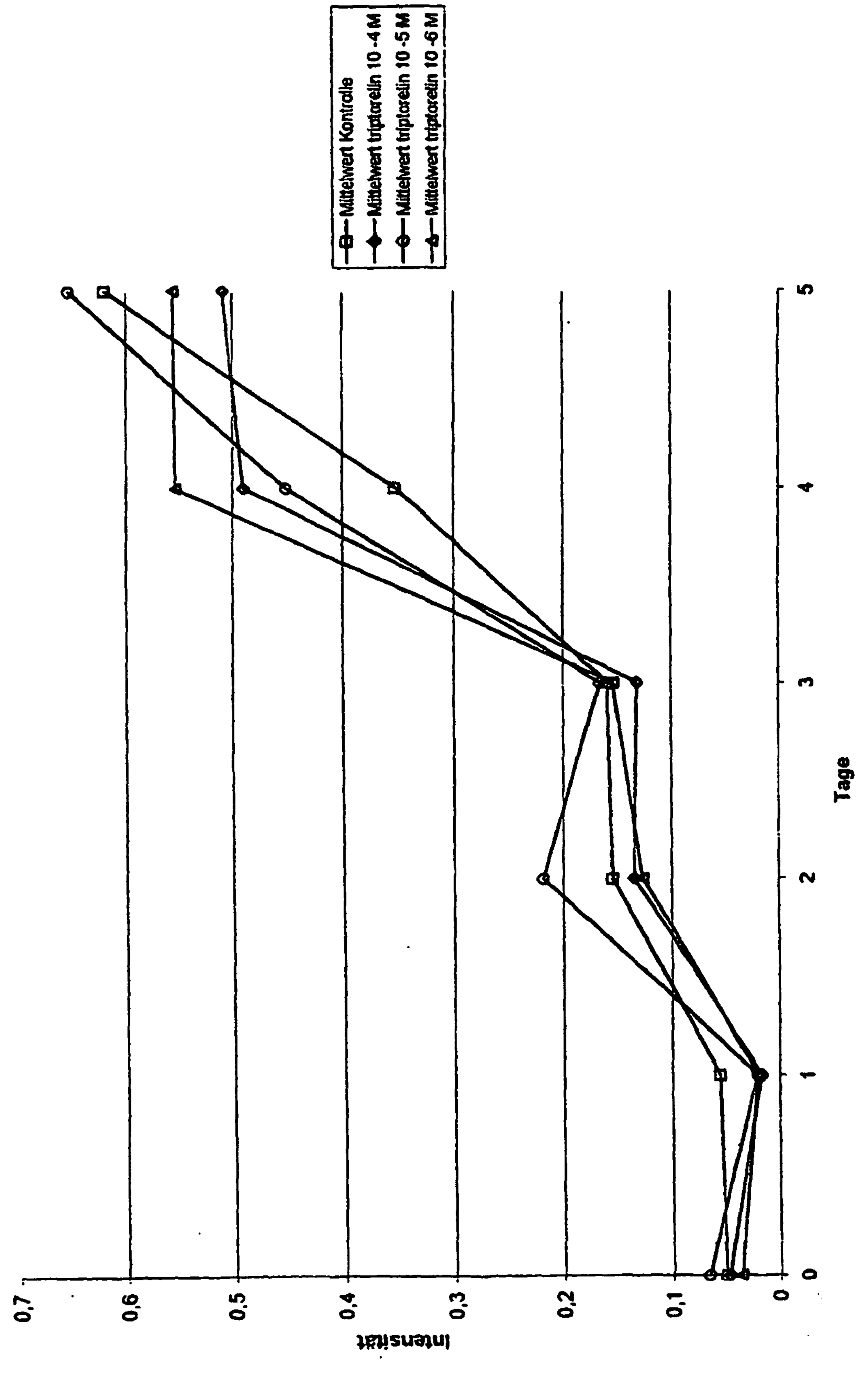

Figur 3 LHRH Hormon

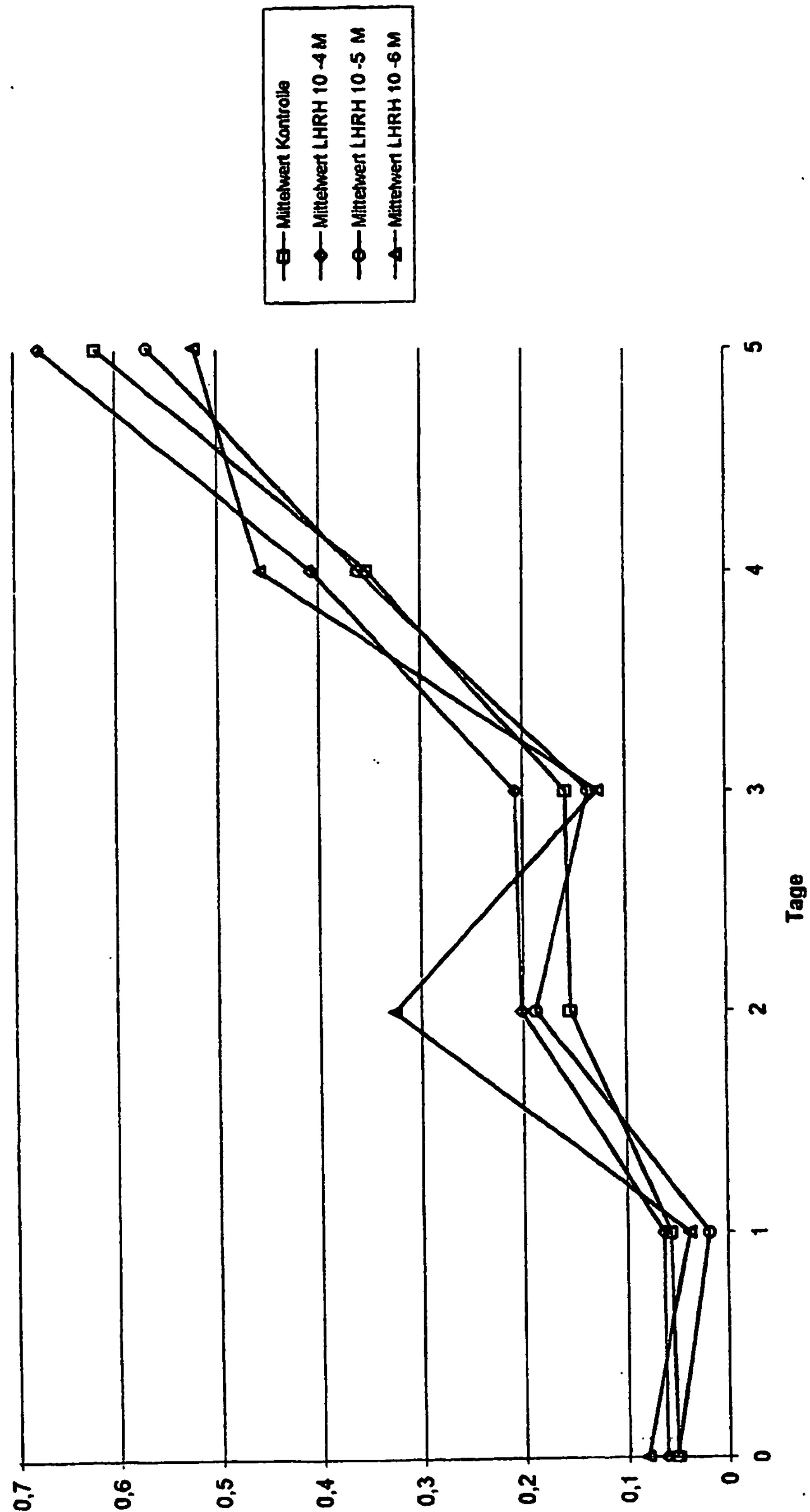

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5480969 A
- GB 2246782 B
- US 5198533 A
- US 444 A
- US 9911004 B


### In der Beschreibung aufgeführte Nicht-Patentliteratur


- **GONZALEZ-BARCENA et al.** *The Prostate,* 1994, vol. 24, 84-92
- **EMONS ; SCHALLY.** *Human Reproduction Update,* 1994, vol. 9 (7), 1364-1379
- **EMONS ; SCHALLY.** *Human Reproduction Update,* 1994, vol. 9, 1364-1379
- **JONAT et al.** *European Journal of Cancer,* 1995, vol. 31A, 137-142
- **RUSSEL-JONES et al.** *Bioconjugate Chem.,* 1995, vol. 6, 34-42
- **TAO et al.** *Neuropeptides,* 1991, vol. 20, 125-131
- **NAOR, Z. ; SCHACHAM, SH. ; HARRIS, D. ; SEGER, R. ; REISS, N.** Signal Transduction of the Gonadotropin Releasing Hormone (GnRH) Receptor: Cross-Talk of Calcium, Protein Kinase C (PKC), and Arachnoidonic Acid. *Cellular and Molecular Neurobiology,* 1995, vol. 15, 527-545
- **ABDILNOUR, G. ; BOURNE, G.A.** Adenosine 3',5'-cyclic mono-phosphate and the self-priming effect of gonadotrophin-releasing hormone. *Molecular and Cellular Endocrinology,* 1995, vol. 107, 1-7
- **ALEXANDER, J.P. ; KLIBANSKI, A.** Gonadotropin-releasing Hormone Receptor mRNA Expression by Human Pituitary Tumors In Vitro. *Journal of Clinical Investigation,* 1994, vol. 93, 2332-2339
- **MAHACHOKLERTWATTANA, P. ; KAPLAN, S.L. ; GRUMBACH, M.M.** The Luteinizing-Hormone-Releasing Hormone-Secreting Hypothalamic Hamartoma Is a Congenital Malformation: Natural History. *Journal of Clinical Endocrinology and Metabolism,* 1993, vol. 77, 118-125
- **SHAMAMIAN et al.** *Cancer Immunol. Immunother.,* 1994, vol. 39, 73-83
- **FLORENES et al.** *Cancer Research,* 1994, vol. 54, 354-356
- **BURG et al.** *Deutsches,* 1997, vol. 94, 890-895
- **TACHIBANA et al.** *J. of Neurosurgery,* 1994, vol. 80, 79-84
- **PUCHNER et al.** *Zentralblatt für Neurochirurgie,* 1996, 47
- **POLLACK et al.** The Efficacy of Tamoxifen as an anti-proliferative Agent in vitro for Benign and Malignant Pediatric Glial Tumors, Pediatr. *Neurosurgery,* 1995, vol. 22, 281-288
- **PAOLETTI et al.** Characteristics and biological role of steroid hormone receptors in neuroepithelial tumors. *J. Neurosurgery,* 1990, vol. 73, 736-742
- **BAUMANN, K. et al.** *Breast Cancer Research Treatment,* 1993, vol. 25, 37-46
- **BARRERA, C ; BANKS, W.A. ; FASOLD, M.B. ; KASTIN, A.J.** Effects of Various Reproductive Hormones on the Penetration of LHRH Across the Blood-Brain Barrier. *Pharmacology, Biochemistry & Behaviour,* 1991, vol. 41, 255-257
- **FLACK et al.** Oral Gossypol in the Treatment of Metastatic Adrenal Cancer. *J. Endocrinol. Metab.,* 1993, vol. 76, 1019-1024
- **LISSONI et al.** Increased Survival Time in Brain Glioblastomas by a Radioneuroendocrine Strategy with Radiotherapy plus Melatonin Compared to Radiotherapy Alone. *Oncology,* 1996, vol. 53, 43-46
- **HUNTER et al.** *Europ. J. Surg. Oncology,* 1993, 242-249
- **IRMER et al.** *Cancer Research,* 1995, vol. 55, 817-822
- **PALYI et al.** *Cancer Delection and Prevention,* 1996, vol. 20, 146-152
- **PATI et al.** *Endocrinology,* 1995, vol. 136, 75-84
- **JONAT et al.** *European J. of Cancer,* 1995, vol. 31A, 137-142
- **CONSTAM et al.** *J. Immunology,* 1992, vol. 148, 1404-1410
- **MELCANGI et al.** *Endocrinology,* 1995, vol. 136, 679-686
- **STOCKHAMMER et al.** *J. of Neurosurgery,* 1995, vol. 83, 672-681
- **JENNINGS et al.** *Hum. Pathol.,* 1994, vol. 25, 464-475

- **BIZIK et al.** *J. Cell Biochem.,* 1996, vol. 62, 113-122
- **VAN BELLE et al.** *Am. J. Pathol.,* 1996, vol. 148, 1887-1894
- **RABKIN et al.** *The New England Journal of Medecine,* 1996, vol. 14, 988-993
- **RUDOLPH, P. et al.** *Am. J. Surg. Pathol.,* 1997, vol. 21 (7), 791-800
- **BOYLE-WASH et al.** Effect of glycoprotein and protein hormones on human meningioma cell proliferation in vitro. *Journal of Endocrinology,* 1995, vol. 145, 155-161
- **ALBINI et al.** The beta-core Fragment of human chorionic gonadotropin inhibits growth of Kaposi sarcoma-derived cells and an new immortalized Kaposi sarcoma cell line. *AIDS,* 1997, vol. 11 (6), 713-721
- **GILL et al.** The effects of preparations of human chorionic gonadotropin on aids-related Kaposi Sarcoma. *The New England Journal of Medicine,* 1996, vol. 335 (17), 1261-1269
- **LIN et al.** *J. Clin. Endocrinol. Metab.,* 1995, vol. 80, 580-585
- **LISSONI.** *European Urology,* 1997, vol. 31, 178-181
- **REGELSON, W. ; PIERPAOLI, W.** *Cancer Invest.,* 1987, vol. 5, 379-385
- **MOTTA et al.** *J. Steroid Biochem. Molec. Biol.,* 1996, vol. 56, 107-11
- **RAO et al.** *Peptides,* 1996, vol. 17, 179-181
- **FATTINGER et al.** *Am. J. Physiol.,* 1996, vol. 271, E775-E787
- **EMONS, G. et al.** *Cancer Research,* 1993, vol. 53, 5439-5446
- **PINSKI et al.** *Cancer Research,* 1994, vol. 54, 5895-5901
- **PARKASH et al.** *New England Journal of Medicine,* 1996, vol. 335 (17), 1261-1269
- **KAKAR, S. et al.** *Biochem. Biophys. Res. Comm.,* 1992, 289-295
- **LEUNG, P.C.K.** *Biological Signals,* 1996, vol. 5, 63-69
- **LIN, L. et al.** *J. Clinical Endocrinol. Metabolism,* 1995, vol. 80 (2), 581-584
- **PARKASH et al.** *New England Journal of Medecine,* 1996, vol. 335 (17), 1261-1269
- **STOCKHAMMER et al.** *J. Neurosurgery,* 1995, vol. 83, 672-681
- **BOYLE-WASH, E. et al.** *Journal of Endocrinology,* 1995, vol. 145, 155-161
- **EMONS et al.** *Cancer Research,* 1993, vol. 53, 539-544
- **KARANDE, A.A. et al.** *Mol. Cell. Endocrinol.,* 1995, vol. 114 (1-2), 51-56
- **ACKERMANN, R. C. et al.** *Cancer Letters,* 1994, vol. 81, 177-184
- **KLIJN, J.G.M. et al.** *The Lancet,* 1982, vol. 19, 12143-1214
- **POLLACK et al.** *Pediatr. Neurosurgery,* 1995, vol. 22, 281-288
- **SPOSTO, R. et al.** *J. Neurooncology,* 1989, vol. 7, 165-177
- **KIRBY, S. et al.** *J. Natl. Cancer Institute,* 1995, vol. 87, 1884-1888
- **LÜ, H.Q. et al.** *Journal of Cancer Research and Clinical Oncology,* 1996, vol. 122, 335-342
- **LIMONTA et al.** *J. Clin. Endocrinol. Metab.,* 1993, vol. 76, 839-845